# EUROPEAN PATENT APPLICATION

(11) **EP 4 431 092 A1**
(43) Date of publication of application: **18.09.2024**
(21) Application number: 23162142.6
(22) Date of filing: 15.03.2023
(51) Int. Cl.: A61K 31/23, A61P 19/02, A61P 37/00, A23L 33/00, A61K 36/00, A61K 45/06

(54) **COMPOSITION FOR USE IN THE THERAPEUTIC TREATMENT OF RHEUMATOID ARTHRITIS**

(71) Applicant: Dr. Schär S.P.A., 39014 Postal (IT)
(72) Inventor: Heidt, Christina, 56235 Ransbach-Baumbach (DE); Zanon, Patrizia, 33043 Cividale del Friuli (IT); Polenghi, Ombretta, 34136 Trieste (IT); Barban, Fabio, 33100 Udine (IT); Cerne, Virna Lucia, 34011 Duino-Aurisina (IT)
(74) Representative: Petraz, Gilberto Luigi

(57) **Abstract**

Embodiments of the present disclosure relate to the field of therapeutic treatment of diseases mediate by immune and inflammatory components, in particular Rheumatoid arthritis (RA) and more particularly, to the use of medium chain triglycerides (MCT) and optionally dietary fibers, for use in the therapeutic treatment of said diseases, in particular RA.

## Description

### FIELD OF THE INVENTION

Embodiments of the present disclosure relate to the field of therapeutic treatment of diseases mediate by immune and inflammatory components, in particular Rheumatoid arthritis (RA) and more particularly, to the use of medium chain triglycerides (MCT) and optionally dietary fibers, for use in the therapeutic treatment of said diseases, in particular RA.

In some further embodiments, said disease may be one of Systemic Lupus Erythematosus (SLE), Osteoarthritis (OA) and Spondyloarthritis (SpA).

### BACKGROUND OF THE INVENTION

### Rheumatoid arthritis

Rheumatoid arthritis (RA) is among the highest occurring chronic inflammatory diseases and is characterized by high joint inflammation leading to chronic pain and progressive joint damage. With patients experiencing a progressive reduction in musculoskeletal functionality and quality of life and an increased risk of comorbidities (e.g. cardiovascular events), RA also leads to extra-joint and systemic manifestations that make the pathology highly invalidating. In order to reduce RA's disease burden on patients, it is then of key importance that RA patients are diagnosed early and that the pharmacological therapy is started promptly; nevertheless, in the medium-long term, innovative therapeutic strategies to control inflammation are strongly needed (Josef S. Smolen et al., 2016).

RA incidence is 0,5-1% of general population, with a higher incidence in women, with a female- to- male sex ratio ranging from 4:1 in younger segments of the population to less than 2:1 in older populations with the disease. Available prevalence estimates for RA indicate that the disease is more common in developed countries and in urban settings than in developing countries and rural settings (Finckh et al., 2022). The overall likelihood of an individual developing RA increases with age, but it is most common to develop the pathology between 40-60 years of age.

50 to 70% of patients present at diagnosis autoantibodies against citrullinated peptides (ACAPs) and autoantibodies against IgG (defined rheumatoid factor [RAs]), which represent the main marker of pathology autoimmunity. It is postulated that autoimmune response originates in the lungs and then moves via the circulatory system to the joints, where a cascade of pro-inflammatory reactions occurs prior to the beginning of joint chronic inflammation.

There is a strong correlation between specific autoantibodies and genetic markers, suggesting that the pathology has an important genetic component. However, pathology progression is also linked to environmental factors, as for instance smoke, low socioeconomic status and low education. An association between periodontal infection and RA onset has been proven. Last but not least, there is growing interest in understanding the relationship between intestinal dysbiosis and risk and progression of RA.

All these evidences suggest that RA is probably due to various damages, and an initial combination of environmental and stochastic events that occurs in a genetically predisposed subjects can lead to a defect in immunologic tolerance towards autologous proteins. These factors, probably in relation with an infection, trigger an autoimmune reaction mediated by T cells (adaptive immunity) that is first localized at the synovia membrane in joints before expanding to other districts (bones, cardiovascular system) (Josef S. Smolen et al., 2016). Clinical manifestation of the pathology is characterized by slow development of signs and symptoms, as for instance stiffness of one or more joints, usually accompanied by pain on movement and by tenderness in the joint. Flares and number of interested joints increase with the progression of inflammatory processes, and other nonspecific systemic symptoms (e.g. fatigue, malaise, depression) as well as related comorbidities (e.g. cardiopulmonary disease) may occur. Disease progression and activity correlates with increasing values of scoring scales, ranging from remission to high disease activity.

### Pharmacological therapy and related side effects

For early diagnosed patients, the main goal of therapeutic treatment is remission, i.e. a state of no or very little inflammatory disease activity, while for patients with prolonged symptoms the goal is achieving a low pathology activity level. RA treatment is based on a periodic evaluation of disease activity that allows to adapt pharmacological intervention to achieve the reversal of inflammation, the main cause of joint damage, disability and comorbidity, following a "treat to target" approach. Main aim is significant disease reduction by 3 months from therapy start, because this maximizes the probability to have remission by 6 months from therapy start. If this goal is not achieved, the treatment is re-evaluated.

Main drugs used to treat RA are
- DMARDs (disease-modifying antirheumatic drugs): the target is inflammation reduction; these drugs are able to reduce tissue damage. Their main side effect is the increase in the risk of severe infections;
- NSAIDs (non-steroidal anti-inflammatory drugs): these drugs, while not capable of reducing pathology progression, significantly reduce pains and j oint infiltration;
- Glucocorticoids: these drugs promptly reduce symptoms and joint damage but they are associated with severe side effects, e.g.
cardiovascular diseases, infections, gastro-intestinal diseases, others.

Once the therapeutic target is reached, drug dosage should be progressively decreased till elimination. Nevertheless, in a high proportion of patients, RA resumes again after drug cessation, causing reduction both in joint functionality and quality of life, and prompting the restoration of the pharmacological treatment. Furthermore, RA patients often remain dependent to drug treatment to keep pathology under control, with linked higher incidence of side effects such as increase in body weight and insulin resistance that correlate with augmented risk of comorbidities.

Considering all the limits of pharmacological treatments, patients very often ask for a diet adaptation to reduce symptoms. Diet effects could be linked to two major action mechanisms: inflammation reduction (mediated by targeting cells or molecules directly involved in inflammation or through antioxidant effect) or comorbidities reduction, as obesity, diabetes, metabolic syndrome (Rondanelli et al., 2021).

### Disease activity indexes

Measuring disease activity is extremely important to evaluate the efficacy of treatments and to monitor disease progression. The disease activity indexes routinely used in clinical practice are based on self-reported information from patient combined with laboratory testing of specific parameters (e.g. hematic C reactive protein) and eventually evaluation by rheumatologist. These indexes allow to allocate patients to subgroups corresponding to different pathology stages (high, moderate, low disease activity or in remission), and give an idea on physical function reduction and joint damage progression. As there is no cure for RA, the aim of the different treatments is to keep disease stable and low, which in turn translates into stable and low (SDAI < 3,3, CDAI < 2,8 or Boolean based evaluation ≤ 1 (Josef S. Smolen et al., 2016)) indexes.

A brief description of two of the indexes mainly used in clinical praxis and clinical studies evaluating nutritional interventions in RA patients follows (Anderson et al., 2011):
- SDAI (Simplified Disease Activity Index)(J. S. Smolen et al., 2003): this index includes the following parameters: i) swollen joint count and tender joint count, ii)self-reported disease activity by patient (measured on a visual analog scale - VAS), iii)disease activity evaluation by clinician (measured on a visual analog scale - VAS), iv) C reactive protein (CrP) blood measurement. A possible interpretation of clinically relevant SDAI variation in nutrition interventions in RA patients with stable pathology is at least one third SDAI reduction (reduction of 3,25 points) compared with minimal clinically relevant SDAI reduction when starting pharmacological treatment in patient with high disease activity (reduction of 13 points) (Edefonti et al., 2020).
- DAS-28 (Disease Activity Score with 28 joint counts)(Prevoo et al., 1995): this index is composed by i)swollen joint count and tender joint count, ii) erythrocyte sedimentation rate (ESR) value, iii)and self-reported disease activity by patient (measured on a visual analog scale
- VAS). A change in 1,2 points in this index is considered clinically significant.

### Microbiome alterations in RA

The connection between microbiota alterations and manifestation and progression of RA has been analyzed in depth, although not all mechanisms and cause-effect relations have been fully explained. New findings obtained via DNA-sequence-based analysis of gut microorganisms indicate that the microorganisms could be an important environmental factor for the development and manifestation of RA. In fact, it has been shown that the microbiome of the intestine, mouth and tracheobronchial system is altered in patients with rheumatoid arthritis (dysbiosis) (Lucchino et al., 2019). As a consequence, the dysbiosis alters the normal homeostasis between microbiota and intestine epithelial cells thus leading to pathology signs as leaky gut.

### RA as a model auto-immune disease

Despite being characterized by a different etiology, RA shares important pathophysiological mechanisms with other diseases, such as Systemic Lupus Erythematosus (SLE), Osteoarthritis (OA) and Spondyloarthritis (SpA).

In fact, SLE has been recognized as a major autoimmune disease for a long time and its immune and inflammatory cascades are mediated by actors - such as Treg cell and inflammasome (e.g. AIM2-inflammasome) - active also in RA (Uresti-Rivera & García-Hernández, 2022). SpA and OA, once thought to be the result of solely mechanical "wear and tear" of cartilages, have been shown to be mediated by immune and inflammatory components (Miller et al., 2020, Ritchlin & Adamopoulos, 2021) similarly to RA. For example, the role of Tumor Necrosis Factor alpha (TNF-alpha), a pro-inflammatory cytokine, is involved in both the initiation and progression of both RA and SpA. (Feldmann, 2002, Braun & Sieper, 2002).

Recent papers have indicated that components of the pathophysiological mechanism of RA, OA, SLE and SpA could be mediated by microbiome modifications in several compartments (e.g. gut and oral compartments), leading to altered inflammatory and immune reactions (Ruff et al., 2020, So & Tam, 2020). The similarity between these diseases is also corroborated by the fact that some medicines are approved for use both in RA and SLE (e.g. methotrexate and hydroxychloroquine).

In the light of these similarities, and bearing in mind the highest incidence for RA than for SLE, OA and SpA, RA is proposed to serve as a model for diseases - such as SLE, OA and SpA - characterized by strong immune and inflammatory pathophysiological components.

### Nutritional interventions in RA

A number of clinical studies focusing on various dietary interventions in patients with RA demonstrated some beneficial effects on pathology symptoms. The results of the different trials led to the development of an ideal dietary pyramid to be followed by patients with RA accompanied by personalized micronutrients supplementation (e.g. vitamin D and omega 3) and avoidance of salt and sugar (Rondanelli et al., 2021). However, there is a limitation in these nutritional indications: there is low number of randomized clinical trials among those at the basis of the pyramid, therefore a strong efficacy demonstration of the intervention is missing (Rondanelli et al., 2021).

The most studied dietary interventions in patients with RA are supplementations with high doses of omega 3 or vitamin D3, or the use of Mediterranean diet, vegetarian/vegan diet, or intermittent fasting for short periods. The results of these dietary intervention were promising enough to increase the interest in counseling RA patients also from a nutritional point of view, however the limitation of the studies, such as high inter-individual variability or transient symptoms reduction, did not allow for the inclusion of these interventions in official treatment guidelines.

In particular:
- Omega 3 and vitamin supplementation is easy to integrate in the diet of RA patients but only reduces patient self-reported pathology parameters;
- Vegetarian/vegan diet induces very individualized responses and mainly depend on food intolerances/allergies even if results are promising;
- Mediterranean diet improves subjective perception of the pathology, but there is not a consensus on the improvement of hematic markers of pathology;
- Fasting, more or less prolonged, gives promising results, but it is difficult to be followed for prolonged periods of time.

Generally speaking, the analysis of 70 clinical studies focused on the above-mentioned nutritional interventions showed a bigger improvement in self-reported subjective pathology indexes rather than objective indexes (Philippou et al., 2021).

### Ketogenic diet and MCT in RA

Primary energy source in human body derives from glucose metabolism, however there is an alternative possible energy source coming from fat utilization. In particular, under certain conditions (e.g. fasting, strenuous exercise), fats can be used as primary source of energy via a process called ketogenesis. In particular, fatty acids mobilized from adipose tissue are converted in ketone bodies, the most abundant being beta hydroxybutyrate (BHB), in the liver. Ketone bodies are then transported to other tissues to serve as cell fuel.

Ketosis, i.e. the increase of hematic ketone bodies, can also be reached thanks to ketogenic diet (KD), a diet rich in fats and with reduced carbohydrate intake. It is well known that medium chain triglycerides (MCT) are among the most ketogenic fats that can be used in KD because they lead to a fast and high increase in ketones.

KD may exert anti-inflammatory effects through three main mechanisms: i) insulin reduction due to the low carbohydrates intake, ii) anti-inflammatory effects of BHB and iii) glucagon increase, each of these mechanism linked to various anti-inflammatory pathways. In addition to these mechanisms, KD contributes to a body weight reduction, which in turn causes a beneficial reduction in background inflammation associated with overweight (Ciaffi et al., 2021).

There is a limited number of clinical trials analyzing KD or intermittent fasting in patients with RA; the results of these trials show that there is still the need to proof the strength of KD in RA in population-based and adequately powered studies (Ciaffi et al., 2021). Nevertheless, document WO 2016/210405 A2 discloses the use of a variety of natural anti-inflammatory compounds, namely epigallochatechin-3-gallate, curcumin and glucosinolates, with optionally the use of a low carbohydrate diet (ketogenic diet, modified ketogenic diet or Akins-like diet) or MCT for the treatment of inflammatory diseases. However, such document discloses the reduction in proinflammatory cytokines thanks to the natural anti-inflammatory compounds mentioned therein, while no mention is made if MCT alone may have a role in anti-inflammatory process.

KD is used since 1920s in the treatment of refractory (i.e. pharmaco-resistant) epilepsy and, most recently, has gained importance in weight loss strategies, being included in official guidelines and clinical practice. Nevertheless, compliance to KD can be difficult to achieve due to different reasons, e.g. severe side effects (hyperlipidemia, exacerbation of gastrointestinal reflux, kidney stones, others), psychosocial factors or restrictiveness of the diet (Ye et al., 2015). For this reasons, variants of the KD, as modified Atkins diet or MCT diet, have been introduced, reaching higher compliance percentages but reducing the effectiveness of the intervention, at least in the field of refractory epilepsy in adults (Ye et al., 2015).

The effect of KD or MCT supplementation on the concentration of ketone bodies in the blood can be easily determined by quantifying BHB in peripheral blood samples. After a normal overnight fasting, BHB reaches a blood concentration of 0,1-0,4 mmol/L, while after 1-3 weeks KD, BHB can reach a blood concentration of 0,5-5 mmol/L (Kolb et al., 2021). Blood ketone levels of 0,5-1,5 mmol/L are considered to correspond to a state of mild ketosis.

Even without a KD, the use of MCTs in combination with a standard (i.e. nonketogenic) diet causes a measurably increased ketonemia and some of the physiological effects - such as increase in brain-derived neurotrophic factor (BDNF) - seen in subjects following a KD are indeed replicated (Norgren et al., 2020). Starting from these considerations, the Applicant recognized the importance of studying whether, in certain condition, the benefits induced by KD could be achieved by supplementation of MCTs to a standard diet, thus achieving higher compliance while reducing side effects.

### Fibers and RA

Dietary fibers are carbohydrate polymers which are not hydrolyzed by endogenous enzymes in small intestine. Dietary fibers are naturally present in foods, especially in fruits and vegetables, but can be selectively added to foods as chemically synthetized or extracted fibers. Fibers are generally categorized in soluble and insoluble fibers according to their capacity to be dispersed in a particular aqueous solution; in addition to this, fibers have particular characteristics according to their chain length, chain structure (linear or branched), capacity to form viscous gels or to trap water. All these characteristics translate into specific effects in humans, ranging from fibers' capacity to lower cholesterol, to improve stool formation, and to be used as an energy source by the gut microbiota. For instance, soluble dietary fibers form viscous gels that may increase digesta transit time, slowing glucose absorption, while non-viscous soluble fibers primarily act as substrate for microbial fermentation, hence being an energy source for bacteria thanks to fiber metabolization to SCFAs (short chain fatty acids) (JR & W, 2016). Insoluble dietary fibers may exert different effects, such as stool softening thanks to their water trapping capacity. Even though it is not their primarily effect because they are not usually fermented, some insoluble fibers can also be metabolized by gut microbiota (McRorie & McKeown, 2017).

Various clinical studies aimed at studying the effect of the dietary supplementation of a specific fiber on the microbiota of RA patients. In particular, two studies analyzed the effects of high fiber supplementation (15 g or 30 g fibers/ day for up to 28 days) on the microbiota in RA patients (Häger et al., 2019)(Dürholz et al., 2020). These clinical studies showed a significant increase in Treg cells, an improvement in some pathology indexes (Short Form 36 Health survey and Physical functioning, Health assessment questionnaire disability index), an improvement of hematic markers of intestine inflammation and intestinal barrier integrity (Häger et al., 2019), an increase in short chain fatty acids (SCFA) and an increase in pro-inflammatory interleukins (Dürholz et al., 2020). Despite all these findings, these studies also showed that the evidences were not sufficient to definitively clarify the role of fibers in RA, and called for additional studies with larger cohorts and longer intervention duration to be performed.

Moreover, document WO 2012/146773 A1 discloses the use of soluble fibers (FOS,GOS,XOS), provided at a dose of 5 g per day, in combination with antioxidants comprising polyphenols, enzymes, liposoluble vitamins D and/or A and amino acids (in particular glutamine).

### Anti-inflammatory effects of MCT and fibers

MCTs (medium chain triglycerides) are considered to be highly ketogenic because they induce a rapid and high increase in ketone bodies, particularly in beta hydroxybutyrate (BHB). MCT could exert anti-inflammatory effects by acting directly on microbiome or through the production of BHB, which in turn acts as anti-inflammatory compound. Various animal studies have shown that MCT fats increase the Bacteroidetes (symbionts) population and reduce Firmicutes and Proteobacteria (pathobionts) and in particular increase the ratio of Bacteroidetes to Firmicutes and Actinobacteria to Proteobacteria (Machate et al., 2020), showing their potential effect in restoring a balanced microbiome. On the other end, BHB is able to interact with specific receptors (in particular G protein coupled receptors GRP 41, 43 and 109a) which activate a cascade of effects including production of anti-inflammatory cytokines or inhibition of proinflammatory pathways.

The human intestinal microbiota is able to ferment fibers into acetate, propionate and butyrate, collectively known as short-chain fatty acids (SCFA). Gram-negative Bacteroides mainly produce acetate and propionate, while Grampositive Firmicutes, such as lactobacilli, streptococci, mycoplasma and clostridia, mainly produce butyrate. SCFA have an influence in both the interaction between microbiota and human intestinal epithelial cells, favoring a healthy environment (e.g. reducing leaky gut signs), and in specific anti-inflammatory pathways, as they target some receptors in common with BHB (e.g. GRP41 and 43 (Z. Ang & Ding, 2016)).

Furthermore, the anti-inflammatory effects of ketosis and/or SCFA could also be mediated by the interaction with T cells, either increase T regulatory cells or reducing proinflammatory Th1/Th17 cells (Q. Y. Ang et al., 2020) (Häger et al., 2019). A similar anti-inflammatory effect was disclosed by document WO 2014/201037 A2, where a probiotic formulation induced a change in Th17, Treg and Th2 cells.

However, the cumulative effect of MCT ad fibers has never been studied in clinical trials in RA patients, thus making the combined effect of these ingredients not known.

### Compliance and duration of nutritional interventions

As reported by several clinical trials, the compliance of patients to a dietary regime completely different from the usual diets is generally low. This is particularly true for interventions such as fasting and ketogenic diet. In the first case, for example, fasting for a defined period showed promising results in reducing RA's pathology indexes, but it is not sustainable for long periods or even not possible in some cases (e.g. in patients with diagnosed gout) (Philippou et al., 2021)

KD is based on a drastic reduction of carbohydrates and an increase in fats, which become major energy source for the body. Even though KD is being routinely used for long periods mainly in patients with pharmaco-resistant epilepsy, the diet is rather difficult to be adhered to by patients and it has to be performed under medical supervision.

Even more simple and easy-to-integrate dietary interventions showed some side effects, resulting in lowered compliance. For instance, in one study with high fiber content added to the standard diet of RA patients, 2 out of 36 patients were induced to leave the study, with additional 3 patients dropping out due to discontinuous adherence to diet or dislike for the high-fiber product (Häger et al., 2019). The low compliance is of particular relevance if one considers that the dietary intervention lasted only 28 days, i.e. a period of time that is extremely limited in comparison to disease duration.

### Micronutrients supplementation in patients with RA

Several lines of evidence support the notion that dietary supplementation of micronutrients is very important for the management of RA. In fact, not only micronutrient deficiencies could arise in RA patients due to their sub-optimal dietary habits, but they also have been correlated with a slowing of RA progression also via reduction in bone erosion (Silva et al., 2016). In addition to this, it is known that some nutrients, such as selenium and omega 3 fatty acids, exert anti-inflammatory actions, which could in turn be beneficial for the management of RA.

In more detail, selenium is included as Selenocysteine in the enzyme Glutathione peroxidase (GPx), a key enzyme for the protection of cells from Reactive Oxygen species. Selenium deficiency causes impairment of glutathione peroxidase, which in turns lead to oxidative stress. More specifically, RA patients have high levels of oxidative stress, and a recent meta-analysis of 14 case-control studies indicated that selenium is significantly lower in RA patients compared to healthy controls (Yu et al., 2016), thus supporting the notion that selenium supplementation could benefit RA patients.

Omega 3 fats are a group of polyunsaturated fatty acids which include octadecatrienoic acid (alfa-linolenic acid), eicosapentaenoic acid (EPA) and docosahexaenoic acid (DHA). Several studies have shown that omega 3 fatty acids have a direct anti-inflammatory effect, which occurs - among other cascades - via reduction of pro-inflammatory cytokines, modulation of T-cell differentiation and also auto-immunity processes. Omega 3 supplementation in RA patients was studied in several clinical trials, which have shown that a supplementation of 2,7 g Omega 3 (EPA+DHA) fats per day supports an improvement in disease signs and symptoms. (Kostoglou-Athanassiou et al., 2020)

There is therefore a need to improve uses, compositions and methods for therapeutic treatment of diseases mediate by immune and inflammatory components, in particular Rheumatoid arthritis (RA) which overcome at least one of the drawbacks in the art.

Furthermore, another purpose of the present invention is to provide compositions and methods for therapeutic treatment of diseases mediate by immune and inflammatory components chosen among Systemic Lupus Erythematosus (SLE), Osteoarthritis (OA) and Spondyloarthritis (SpA).

Various limitations and disadvantages of conventional solutions and technologies will become apparent to one of skill in the art after reviewing the remainder of the present application with reference to the drawings and description of the embodiments which follow, though it should be understood that this description of the related art section is not intended to serve as an admission that the described subject matter is prior art.

### SUMMARY OF THE INVENTION

According to embodiments, medium chain triglycerides (MCT), optionally combined with dietary fibers, for use in therapeutic treatment of diseases mediate by immune and inflammatory components are provided.

According to possible embodiments, combinable with all embodiments described herein, said disease may be selected from any of Rheumatoid arthritis (RA), Systemic Lupus Erythematosus (SLE), Osteoarthritis (OA) and Spondyloarthritis (SpA).

In embodiments, combinable with all embodiments described herein, treatment of said disease comprises inducing ketosis in a subject.

According to further embodiments, a composition comprising MCT and optionally dietary fibers, for use in the therapeutic treatment of diseases mediate by immune and inflammatory components is provided.

In embodiments, combinable with all embodiments described herein, the composition may be free of natural anti-inflammatory compounds chosen among epigallochatechin-3-gallate, curcumin and glucosinolates and/or derivatives thereof (such as glucoraphanin (GRP) and/or sulforaphane (SFN) as found in broccoli sprouts or sprouts of other cruciferous vegetables).

In embodiments, combinable with all embodiments described herein, the composition is suitable for human consumption and can be used to induce ketosis in a subject.

In embodiments, combinable with all embodiments described herein, the composition may comprise a ratio of MCT to dietary fibers, and may further comprise fortifying food ingredients and/or micronutrients, sweeteners, and/or flavoring agents.

In embodiments, which can be combined with all embodiments described herein, dietary fibers comprise insoluble fibers and soluble fibers.

The composition can be administered in various forms, including as a liquid, bakery product, or other food with a fat-based or water-containing filling.

According to embodiments, which can be combined with all embodiments described herein, said disease is RA.

According to embodiments, which can be combined with all embodiments described herein, medium chain triglycerides (MCT), optionally combined with dietary fibers, for use in therapeutic treatment of RA are provided.

According to further embodiments, a composition comprising MCT and optionally dietary fibers, for use in the therapeutic treatment of RA is provided.

These and other features, aspects and advantages of the present disclosure will become better understood with reference to the following description, the drawings and appended claims. The drawings, which are incorporated in and constitute a part of this specification, illustrate embodiments of the present subject matter and, together with the description, serve to explain the principles of the disclosure.

The various aspects and features described in the present disclosure can be applied, individually, wherever possible. These individual aspects, for instance the aspects and features described in the attached dependent claims, can be made subject of divisional patent applications.

It is noted that anything found to be already known during the patenting process is understood not to be claimed and to be the subject of a disclaimer.

### DETAILED DESCRIPTION OF THE EMBODIMENTS

Reference will now be made in detail to the various embodiments of the invention, one or more examples of which are illustrated in the figures. Each example is provided by way of explanation of the invention and is not meant as a limitation of the invention. For example, features illustrated or described as part of one embodiment can be used on or in conjunction with other embodiments to yield yet a further embodiment. It is intended that the present invention includes such modifications and variations.

Before describing these embodiments, it shall be clarified that the phraseology and terminology used here is for the purposes of description only, and cannot be considered as limitative.

All the percentages and ratios indicated refer to the weight of the total composition (for example indicated as % wt/wt), unless otherwise indicated. All the measurements are made, unless otherwise indicated, at 25°C and atmospheric pressure. All the temperatures, unless otherwise indicated, are expressed in degrees Centigrade.

All the ranges reported here shall be understood to include the extremes, including those that report an interval "between" two values. Furthermore, all the ranges reported here shall be understood to include and describe the punctual values included therein, and also all the sub-intervals. Moreover, all the ranges are intended as such that the sum of the values comprised therein, in the final composition, gives 100%, in particular considering that the person of skill will know how to choose the values of the ranges so that the sum does not exceed 100%.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. To the extent such publications may set out definitions of a term that conflicts with the explicit or implicit definition of the present disclosure, the definition of the present disclosure controls. In addition, citation or identification of any reference in this application shall not be construed as an admission that such reference is available as prior art to the present invention.

The Applicant for the first time discovered that MCT alone, optionally in combination with dietary fibers, can be effective in the therapeutic treatment of a diseases mediated by immune and inflammatory components.

According to possible embodiments, combinable with all embodiments described herein, said disease may be selected from any of Rheumatoid arthritis (RA), Systemic Lupus Erythematosus (SLE), Osteoarthritis (OA) and Spondyloarthritis (SpA).

In specific embodiments, said disease can be RA. In particular, the cumulative and synergistic effect of MCT and fibers in treatment of RA was for the first time discovered, studied and proved by the Applicant in clinical trials in patients with RA.

Embodiments described herein may further provide a composition comprising MCT and optionally dietary fibers, for use in the therapeutic treatment of said diseases, in particular for instance RA. The composition is suitable for human consumption, and can be used to induce ketosis in a subject, which has been suggested to have therapeutic benefits in said diseases, in particular for instance RA.

In particular, embodiments described herein are based on the discovery that a composition comprising medium chain triglycerides (MCT) and optionally dietary fibers can be used for therapeutic treatment of said diseases, in particular for instance RA, by inducing ketosis in the subject.

Without being bound by theory, the Applicant hypothesizes that a possible mechanism by which MCTs could reduce disease(s) activity is through microbiota-mediated host effects on gut-barrier function and autoimmunity.

In embodiments, the composition according to the present disclosure is free of any natural anti-inflammatory compounds chosen among epigallochatechin-3-gallate, curcumin and glucosinolates.

The composition may comprise a ratio of MCT to dietary fibers, with an insoluble fibers to soluble fibers ratio of 1,5:1 to 3:1 wt/wt. The ratio of MCT to dietary fibers may be in the range of 0,5:1 to 2:1, and in particular, 0,75:1 to 1,5:1 wt/wt.

The MCT may be provided as pure form or as a substance rich in MCT, and in particular said MCT may be in either powder or liquid form. A source of MCT may be e.g. palm fat or coconut fat.

The MCT may comprise a ratio of octanoic acid to decanoic acid of 50:50 to 70:30 wt/wt, in particular 55:45 to 65:35 wt/wt. A possible example is 60:40 wt/wt.

In embodiments, which can be combined with other embodiments described herein, the composition may comprise proteins, carbohydrates, fibers and fat including said MCT.

Additionally, the composition may comprise fortifying food ingredients and/or micronutrients, sweeteners, and/or flavoring agents.

In particular, the composition may comprise fortifying food ingredients and/or micronutrients, in particular including one or more of omega 3, for example docosahexaenoic acid (DHA), eicosapentaenoic acid (EPA), minerals, trace elements, for example selenium, vitamins or a combination thereof. In one embodiment, the composition may comprise sweeteners and/or flavoring agents to improve the palatability of the composition.

In embodiments, which can be combined with other embodiments described herein, the composition may essentially consist of proteins, carbohydrates, fibers, fat including said MCT and optionally one or more of fortifying food ingredients and/or micronutrients, sweeteners and/or flavoring agents.

In embodiments, which can be combined with other embodiments described herein, the composition may essentially consist of fat including said MCT and optionally one or more of fortifying food ingredients and/or micronutrients, sweeteners and/or flavoring agents.

The composition can be administered in various forms, including foods in liquid or solid form, as powder product to be reconstituted with water, bars, bakery products, semi-solid products or liquid products. In possible examples, the composition may be provided as a beverage, mayonnaise, salad dressing, margarine, low fat spread, dairy product, cheese spread, processed cheese, dairy dessert, flavored milk, cream, fermented milk product, cheese, butter, condensed milk product, ice cream mix, soya product, pasteurized liquid egg, bakery product, porridge, breakfast cereals, confectionary product, confectionary bar, chocolate bar, high fat bar, UHT pudding, pasteurized pudding, gel, jelly, yoghurt, or a food with a fat-based or water-containing filling.

In embodiments, which can be combined with other embodiments described herein, the composition may contain MCT up to 100% wt/wt, in particular in a range of 2 to 80% wt/wt, in particular 5 to 60% wt/wt, more in particular 10 to 50% wt/wt, still more in particular 15 to 35% wt/wt. Possible examples are 15%, 20%, 25%, 30%, 35%, 40%, 50%, 55%, 60%, 65%, 70%, 75%, 80%, 85%, 90%, 95%, 100% wt/wt.

In further embodiments, which can be combined with other embodiments described herein, the composition may contain dietary fibers in a range of 2 to 50% wt/wt, in particular 5 to 45% wt/wt, more in particular 10 to 50% wt/wt, still more in particular 15 to 35% wt/wt. A possible example is 30% wt/wt.

In a particular embodiment, a dosage of at least 12g to 60g, in particular 15g to 45g, e.g. 30g, MCT, and optionally of at least 10g to 60g, in particular 15g to 45g, e.g. 30g, of dietary fibers, may be administered per day to the subject in need of the therapeutic treatment of one of said diseases, in particular for instance RA. In embodiments, the dosage of MCT and optionally dietary fibers may depend on the severity of the disease, e.g. RA, symptoms and the response of the subject to the treatment.

In a further embodiment, a liquid composition suitable for human consumption may be provided, comprising a composition according to the present disclosure, reconstituted with an aqueous liquid. The liquid composition may be used as a beverage, mayonnaise, salad dressing, or any other liquid food product.

In yet another embodiment, a bakery product may be provided, comprising a composition according to the present disclosure. The bakery product can be a bread, muffin, pastry, nutrition bar, crispbread or any other baked good.

In a still further embodiment, medium chain triglycerides (MCT) for use in the therapeutic treatment of said diseases, in particular for instance RA, may be provided. The MCT may be used alone or in combination with dietary fibers and other fortifying food ingredients and/or micronutrients, sweeteners and/or flavoring agents, and may be formulated in various food products, such as beverages, mayonnaise, salad dressing, margarine, low fat spread, dairy product, cheese spread, processed cheese, dairy dessert, flavoured milk, cream, fermented milk product, cheese, butter, condensed milk product, ice cream mix, soya product, pasteurised liquid egg, bakery product, porridge, breakfast cereals, confectionary product, confectionary bar, chocolate bar, high fat bar, UHT pudding, pasteurised pudding, gel, jelly, yoghurt, or a food with a fat-based or water-containing filling.

The embodiments described herein thus provide safe and effective composition and method for the therapeutic treatment of said diseases, in particular for instance RA, which utilizes the beneficial effects of MCT and optionally dietary fibers, in particular in inducing ketosis and reducing inflammation and oxidative stress. The embodiments described herein may further provide a wide range of food products and compositions that may be consumed by the subject in a convenient and enjoyable manner, and that may provide additional fortifying food ingredients and/or micronutrients, sweeteners and/or flavoring agents, as well as a wide range of food textures and tastes. The compositions according to the embodiments described herein are easy to prepare, have good palatability, and can be incorporated into various food products, making them a convenient and practical treatment option. The embodiments of the present disclosure also provide an alternative to traditional treatments for the above mentioned diseases, e.g. RA, which may have undesirable side effects.

### EXAMPLES

The Applicant developed some exemplary products for therapeutic treatment of the above mentioned diseases, for instance RA, having a core recipe composed by MCT and a fiber mix (e.g. bamboo, psyllium and maize fiber). Additional exemplary ingredients are proteins, carbohydrates (mainly in form of polyols), sweeteners, aromas and other flavoring substances (e.g. cacao powder) and micronutrients fortification (including omega 3 fatty acids, for example DHA, EPA, minerals, trace elements, for example selenium, and vitamins).

The different product forms are designed by the Applicant to deliver a daily dosage of e.g. 30 g MCT and 30 g fibers, divided in two or more portion per day. The products may have a high fat and fiber (respectively 66-76 % and 11-15 % of energy), a low-to high protein (3-17 % of energy), and a low carbohydrate (2-10 % of energy) content. Carbohydrates are mainly in form of polyols (e.g. erythritol) and sugars are below 2,5 % of the recipe. Fibers are added as a mixture to reach a ratio insoluble to soluble fibers of 3:1. Other examples may be 1,5: 1 or 2,5:1.

In embodiments, combinable with all embodiments described herein, said fibers may include one or more of bamboo fiber, psyllium fiber, maize fiber. In specific embodiments, said fibers may include all of bamboo fiber, psyllium fiber, maize fiber.

One possible product developed by the Applicant is a powder product, called porridge, to be used after reconstitution with water (two exemplary dilution possibilities, 50 ml to obtain a dense porridge, 100 ml or even 150 ml to obtain a less dense product).

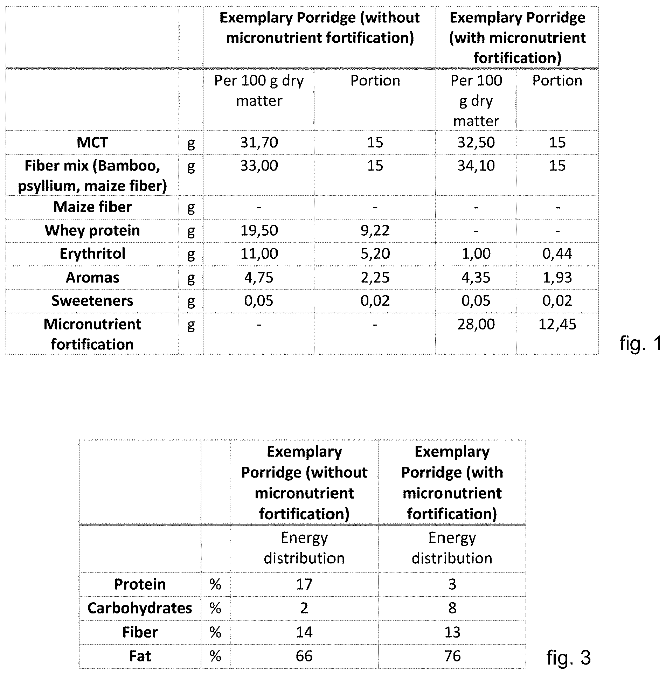

The recipe is shown in the table of Figure 1, with and without micronutrient fortification. Values are expressed per 100 gr dry matter and per portion (47,3 g powder without micronutrient fortification, 44,5 g with micronutrient fortification). In the case of porridge, a portion corresponds to 15 g MCT and 15 g fiber mix.

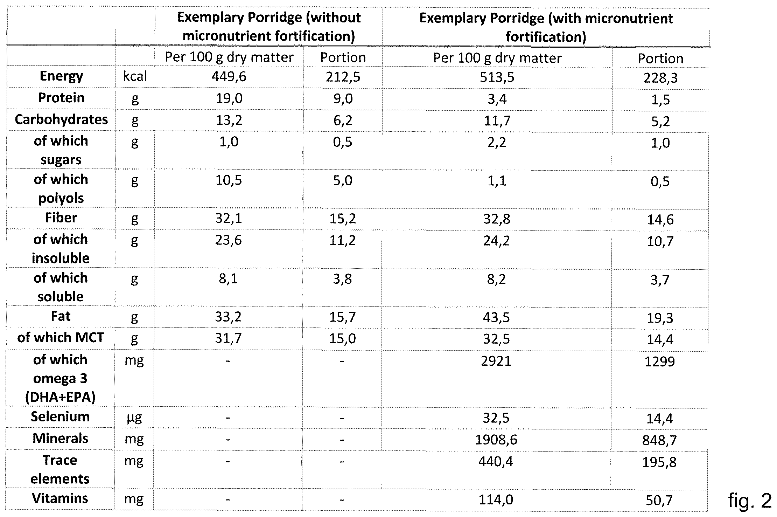

In the table of Figure 2, nutritional values of exemplary porridge, with and without micronutrient fortification, are presented per 100 g dry matter and per portion (47,3 g powder without micronutrient fortification, 44,5 g with micronutrient fortification).

The table of Figure 3 shows energy distribution among macronutrients in the exemplary porridge, corresponding to percentage of each macronutrient to total energy.

Other possible product forms may be baked products, as but not limited to biscuits, crispbread, muffin, bars, bread, or semisolid products, as yogurt or spoonable desserts.

Portion weight may be calculated to reach the target of e.g. 30 g MCT and 30 g fibers per day with two or four servings.

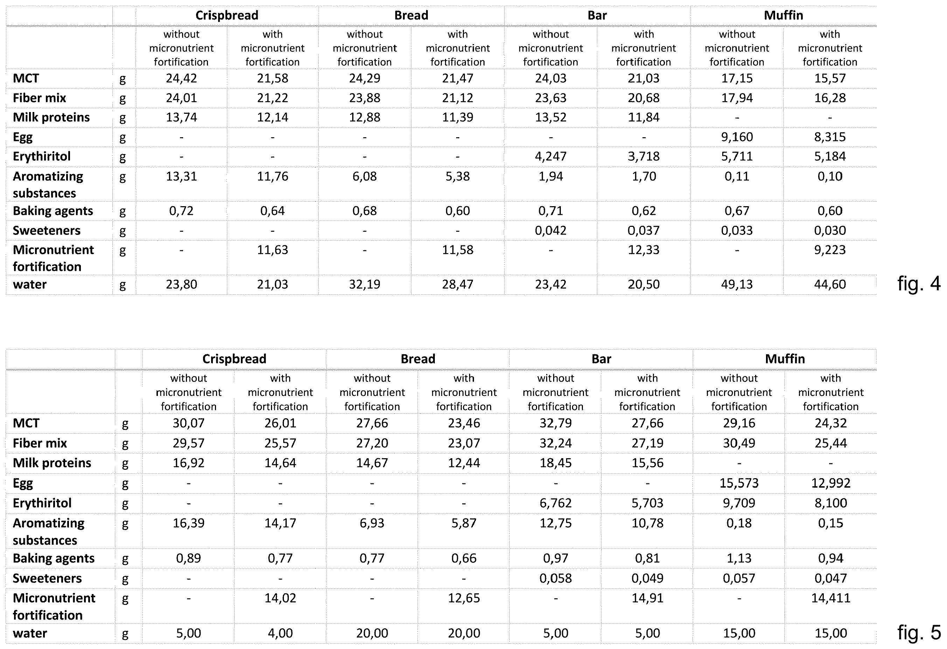

Recipes of the baked products before baking are presented in the table of Figure 4 (all values are expressed per 100 g dough).

Recipes of the baked products after baking are presented in the table of Figure 5 (all values are expressed per 100 g finished product).

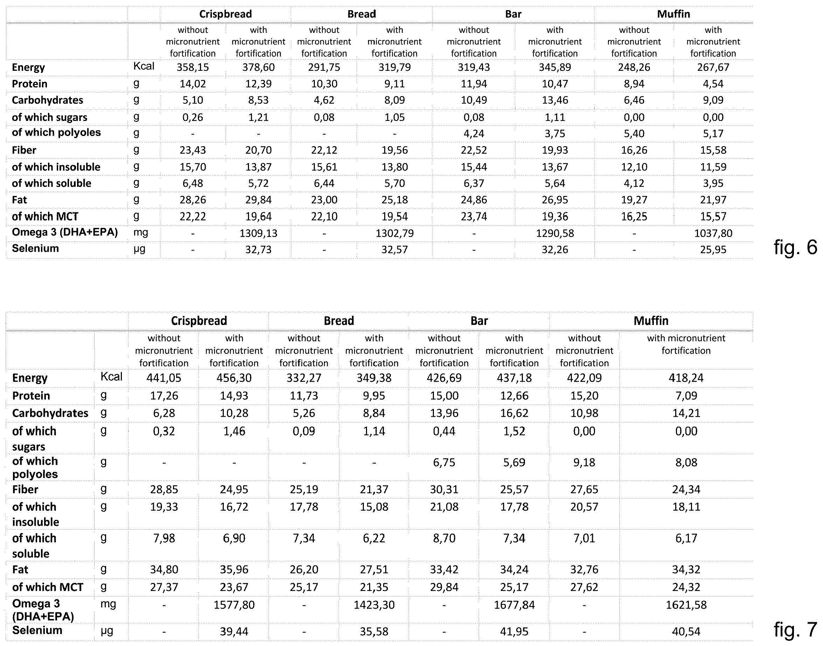

In the table of Figure 6, nutritional values of exemplary baked products before baking, with and without micronutrient fortification, are presented per 100 g dough.

In the table of Figure 7, nutritional values of exemplary baked products after baking, with and without micronutrient fortification, are presented per 100 g finished product.

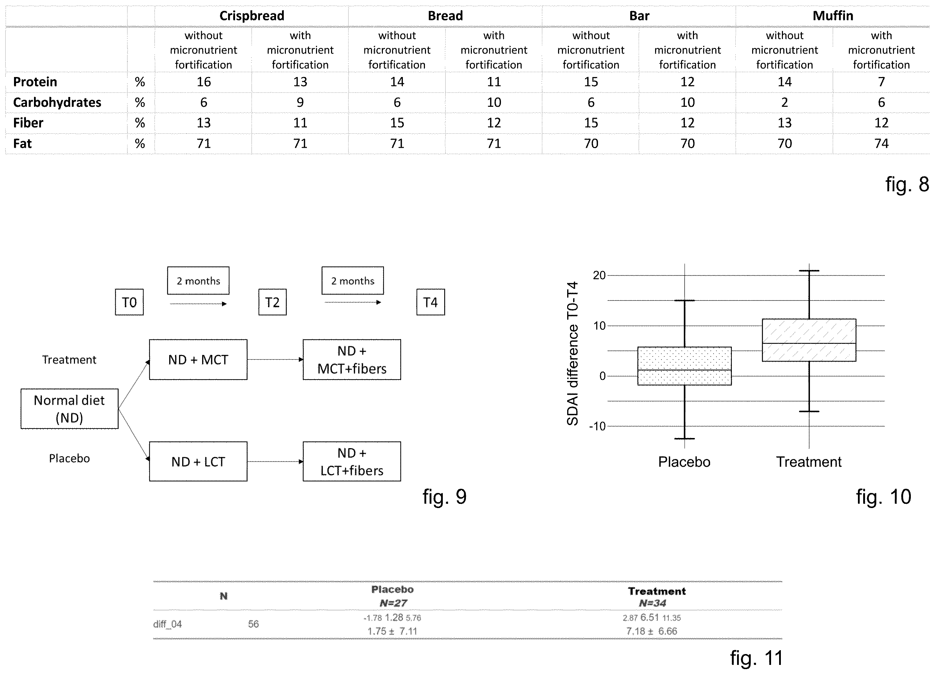

The table of Figure 8 shows energy distribution among macronutrients in baked products, corresponding to percentage of each macronutrient to total energy.

Micronutrient fortification, both in porridge and in baked products, is added to the recipes with the aim to reach specific daily micronutrient supplementation. In particular this might include Omega 3, as sum of EPA and DHA, at a level of 2,7 g/day and selenium in a range from 30 to 100µg/day.

### EXPERIMENTAL DATA

The efficacy of using MCT, optionally combined with dietary fibers, for therapeutic treatment of RA has been assessed according to an intervention prospective study, with randomized, double blind, placebo-controlled design.

RA patients included in the study, aged between 55 and 75 years, where in low to remission stage of RA and under stable medication. 61 subjects were included in the study, and were randomly assigned to the treatment group or the placebo group and followed the dietary interventions for a total of 4 months. The nutritional intervention in the treatment group was to take two portions of porridge per day in addition to the patients' normal diet, reaching a consumption of 30 g MCT from study start (T0) to month 2 (T2), followed by 30 g MCT plus 30 g fibers from month 2 (T2) to month 4 (T4). Similarly, patients in the placebo group received 30 g LCT in form of sunflower oil (T0-T2) followed by 30 g LCT plus 30 g fibers (T2-T4) per day.

Figure 9 is a schematic representation of an example of the dietary intervention.

Primary endpoint of the study was evaluation of SDAI reduction from T0 to T4.

Figure 10 and table of Figure 11 show the difference in SDAI index from T0 to T4 in placebo and treated group.

Figure 10 shows the SDAI index reduction after 4 months dietary intervention. Placebo=30 g LCT/30 g LCT+30 g fibers per day; Treatment=30 g MCT/30 g MCT+30 g fibers per day.

The table of Figure 11 shows the SDAI index reduction after 4 months dietary intervention. Placebo=30 g LCT/30 g LCT+30 g fibers per day; Treatment=30 g MCT/30 g MCT+30 g fibers per day

The comparison of SDAI index reduction from T0 to T4 between the treatment group (median: 6,51) and the placebo group (median: 1,28) resulted in a significant difference (p=0,008048, U-test according to Mann and Whitney or the Wilcoxon test). SDAI index was reduced by 5.23 point more in the treated group if compared to the placebo group. Median SDAI reduction of 6,51 observed in treatment group is even more than one third index reduction in active pathology to be considered clinically relevant, as described by Edefonti. Surprisingly, nutritional intervention with exemplary porridge showed a relevant improvement in clinical symptoms, far beyond the foreseeable combination of MCT and fibers positive aspects on inflammation reduction.

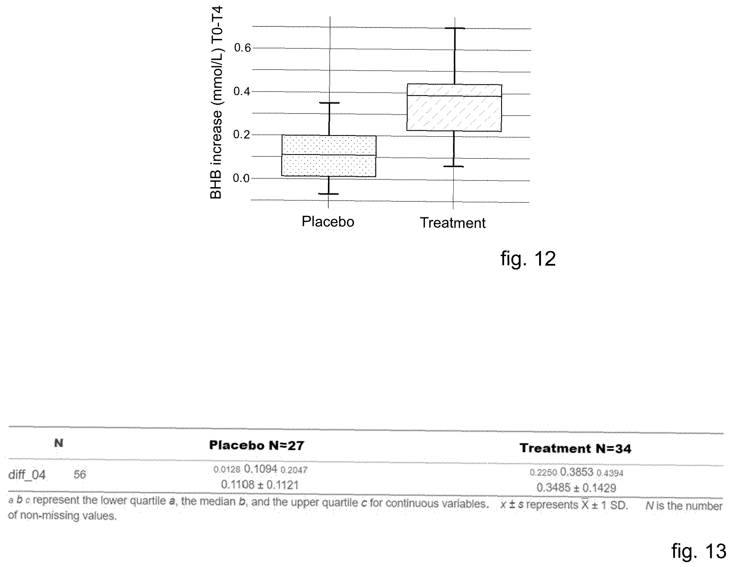

Ketosis level was also analyzed at T0 and T4. Figure 12 and table of Figure 13 show the difference in BHB increase from T0 to T4 in placebo and treated group.

Figure 12 shows the BHB increase after 4 months dietary intervention. Placebo=30 g LCT/30 g LCT+30 g fibers per day; Treatment=30 g MCT/30 g MCT+30 g fibers per day.

The table of Figure 13 shows the BHB increase after 4 months dietary intervention. Placebo=30 g LCT/30 g LCT+30 g fibers per day; Treatment=30 g MCT/30 g MCT+30 g fibers per day

The comparison of ketosis increase from T0 to T4 between the treatment group (median: 0,39 mmol/L) and the placebo group (median: 0,11 mmol/L) showed a statistically significant difference (p<0.001, U-test according to Mann and Whitney or the Wilcoxon test). Ketosis level reached in the treatment group is about 0.49mmol/L BHB, thus considered to be in the range of mild ketosis.

Patients where highly compliant to the dietary intervention and no GI symptoms have been reported.

The results of the intervention prospective study with a randomized, doubleblind, placebo-controlled design demonstrate that the use of MCT, optionally combined with dietary fibers, for therapeutic treatment of RA is effective. The primary endpoint of the study was SDAI reduction from start to end of the intervention, with the treated group (MCT/MCT+fiber) showing a median SDAI reduction of 6,51, significantly higher than the placebo group (LCT/LCT+fiber) with a median SDAI reduction of 1,28. The study also showed that the nutritional intervention according to embodiments described herein was well-tolerated by the patients, with no reported GI symptoms. Overall, these findings show that the use of MCT, optionally combined with dietary fibers, in the treatment of RA is a valuable therapeutic option.

While the foregoing is directed to embodiments of the invention, other and further embodiments of the invention may be devised without departing from the basic scope thereof, and the scope thereof is determined by the claims that follow.

### BIBLIOGRAPHY

Anderson, J. K., Zimmerman, L., Caplan, L., & Michaud, K. (2011). Measures of rheumatoid arthritis disease activity: Patient (PtGA) and Provider (PrGA) Global Assessment of Disease Activity, Disease Activity Score (DAS) and Disease Activity Score with 28-Joint Counts (DAS28), Simplified Disease Activity Index (SDAI), Cl. Arthritis Care and Research. https://doi.org/10.1002/acr.20621
Ang, Q. Y., Alexander, M., Newman, J. C., Tian, Y., Cai, J., Upadhyay, V., Turnbaugh, J. A., Verdin, E., Hall, K. D., Leibel, R. L., Ravussin, E., Rosenbaum, M., Patterson, A. D., & Tumbaugh, P. J. (2020). Ketogenic Diets Alter the Gut Microbiome Resulting in Decreased Intestinal Th17 Cells. Cell, 181(6), 1263-1275.e16. https://doi.org/10.1016/j.cell.2020.04.027
Ang, Z., & Ding, J. L. (2016). GPR41 and GPR43 in obesity and inflammation - Protective or causative? In Frontiers in Immunology. https://doi.org/10.3389/fimmu.2016.00028
Braun, J., & Sieper, J. (2002). Therapy of ankylosing spondylitis and other spondyloarthritides: Established medical treatment, anti-TNF-α therapy and other novel approaches. Arthritis Research, 4(5), 307-321. http s: //do i. org/10.118 6/ar5 92
Ciaffi, J., Mitselman, D., Mancarella, L., Brusi, V., Lisi, L., Ruscitti, P., Cipriani, P., Meliconi, R., Giacomelli, R., Borghi, C., & Ursini, F. (2021). The Effect of Ketogenic Diet on Inflammatory Arthritis and Cardiovascular Health in Rheumatic Conditions: A Mini Review. Frontiers in Medicine, 8(December), 1-9. https://doi.org/10.3389/fmed.2021.792846
Dürholz, K., Hofmann, J., Iljazovic, A., Häger, J., Lucas, S., Sarter, K., Strowig, T., Bang, H., Rech, J., Schett, G., & Zaiss, M. M. (2020). Dietary shortterm fiber interventions in arthritis patients increase systemic scfa levels and regulate inflammation. Nutrients, https://doi.org/10.3390/nu12103207
Edefonti, V., Parpinel, M., Ferraroni, M., Boracchi, P., Schioppo, T., Scotti, I., Ubiali, T., Currenti, W., De Lucia, O., Cutolo, M., Caporali, R., & Ingegnoli, F. (2020). A posteriori dietary patterns and rheumatoid arthritis disease activity: A beneficial role of vegetable and animal unsaturated fatty acids. Nutrients, 12(12), 1-22. https://doi.org/10.3390/nu12123856
Feldmann, M. (2002). Development of anti-TNF therapy for rheumatoid arthritis. In Nature Reviews Immunology. https://doi.org/10.1038/nri802
Finckh, A., Gilbert, B., Hodkinson, B., Bae, S. C., Thomas, R., Deane, K. D., Alpizar-Rodriguez, D., & Lauper, K. (2022). Global epidemiology of rheumatoid arthritis. Nature Reviews Rheumatology, 18(10), 591-602. https://doi.org/10.1038/s41584-022-00827-y
Häger, J., Bang, H., Hagen, M., Frech, M., Träger, P., Sokolova, M. V., Stéen, U., Tascilar, K., Sarter, K., Schett, G., Rech, J., & Zaiss, M. M. (2019). The role of dietary fiber in rheumatoid arthritis patients: A feasibility study. Nutrients, 11(10), 1-15. https://doi.org/10.3390/nu11102392
JR, P., & W, Y. (2016). A Review of Physiological Effects of Soluble and Insoluble Dietary Fibers. Journal of Nutrition & Food Sciences, 06(02). https://doi.org/10.4172/2155-9600.1000476
Kolb, H., Kempf, K., Röhling, M., Lenzen-Schulte, M., Schloot, N. C., & Martin, S. (2021). Ketone bodies: from enemy to friend and guardian angel. BMC Medicine, 19(1), 1-15. https://doi.org/10.1186/s12916-021-02185-0
Kostoglou-Athanassiou, I., Athanassiou, L., & Athanassiou, P. (2020). The effect of omega-3 fatty acids on rheumatoid arthritis. Mediterranean Journal of Rheumatology. https://doi.org/10.31138/mjr.31.2.190
Lucchino, B., Spinelli, F. R., Iannuccelli, C., Guzzo, M. P., Conti, F., & Di Franco, M. (2019). Mucosa-environment interactions in the pathogenesis of rheumatoid arthritis. Cells, 8(7). https://doi.org/10.3390/cells8070700
Machate, D. J., Figueiredo, P. S., Marcelino, G., Guimarães, R. de C. A., Hiane, P. A., Bogo, D., Pinheiro, V. A. Z., de Oliveira, L. C. S., & Pott, A. (2020). Fatty acid diets: Regulation of gut microbiota composition and obesity and its related metabolic dysbiosis. International Journal of Molecular Sciences, 21(11), 1-22. https://doi.org/10.3390/ijms21114093
McRorie, J. W., & McKeown, N. M. (2017). Understanding the Physics of Functional Fibers in the Gastrointestinal Tract: An Evidence-Based Approach to Resolving Enduring Misconceptions about Insoluble and Soluble Fiber. Journal of the Academy of Nutrition and Dietetics, 117(2), 251-264. https://doi.org/10.1016/j.jand.2016.09.021
Miller, R. J., Malfait, A. M., & Miller, R. E. (2020). The innate immune response as a mediator of osteoarthritis pain. Osteoarthritis and Cartilage, 28(5), 562-571. https://doi.org/10.1016/j.joca.2019.11.006
Norgren, J., Sindi, S., Sandebring-Matton, A., Kåreholt, I., Daniilidou, M., Akenine, U., Nordin, K., Rosenborg, S., Ngandu, T., & Kivipelto, M. (2020). Ketosis After Intake of Coconut Oil and Caprylic Acid-With and Without Glucose: A Cross-Over Study in Healthy Older Adults. Frontiers in Nutrition, https://doi.org/10.3389/fnut.2020.00040
Philippou, E., Petersson, S. D., Rodomar, C., & Nikiphorou, E. (2021). Rheumatoid arthritis and dietary interventions: Systematic review of clinical trials. Nutrition Reviews, 79(4), 410-428. https: //doi. org/ 10.1093/nutrit/nuaa03 3
Prevoo, M. L. L., Van'T Hof, M. A., Kuper, H. H., Van Leeuwen, M. A., Van De Putte, L. B. A., & Van Riel, P. L. C. M. (1995). Modified disease activity scores that include twenty-eight-joint counts development and validation in a prospective longitudinal study of patients with rheumatoid arthritis. Arthritis & Rheumatism. https://doi.org/10.1002/art.1780380107
Ritchlin, C., & Adamopoulos, I. E. (2021). Axial spondyloarthritis: New advances in diagnosis and management. In The BMJ. https://doi.org/10.1136/bmj.m4447
Rondanelli, M., Perdoni, F., Peroni, G., Caporali, R., Gasparri, C., Riva, A., Petrangolini, G., Faliva, M. A., Infantino, V., Naso, M., Perna, S., & Rigon, C. (2021). Ideal food pyramid for patients with rheumatoid arthritis: A narrative review. Clinical Nutrition, 40(3), 661-689. https://doi.org/10.1016/j.clnu.2020.08.020
Ruff, W. E., Greiling, T. M., & Kriegel, M. A. (2020). Host-microbiota interactions in immune-mediated diseases. Nature Reviews Microbiology, 18(9), 521-538. https://doi.org/10.1038/s41579-020-0367-2
Silva, G. B., Reis, B. Z., & Franciscato, S. M. C. (2016). Pathology and Clinical Research Micronutrients Deficiencies in Rheumatoid Arthritis Patients ClinMed. International Journal of Pathology and Clinical Research, 2(1), 1-5.
Smolen, J. S., Breedveld, F. C., Schiff, M. H., Kalden, J. R., Emery, P., Eberl, G., van Riel, P. L., & Tugwell, P. (2003). A simplified disease activity index for rheumatoid arthritis for use in clinical practice. Rheumatology, 42(2), 244-257. https://doi.org/10.1093/rheumatology/keg072
Smolen, Josef S., Aletaha, D., & McInnes, I. B. (2016). Rheumatoid arthritis. The Lancet, 388(10055), 2023-2038. https://doi.org/10.1016/S0140-6736(16)30173-8
So, J., & Tam, L. S. (2020). Gut microbiome and its interaction with immune system in spondyloarthritis. Microorganisms, 8(11), 1-14. https://doi.org/10.3390/microorganisms8111727
Uresti-Rivera, E. E., & García-Hernández, M. H. (2022). AIM2-inflammasome role in systemic lupus erythematous and rheumatoid arthritis. Autoimmunity, 55(7), 443-454. https://doi.org/10.1080/08916934.2022.2103802
Ye, F., Li, X. J., Jiang, W. L., Sun, H. Bin, & Liu, J. (2015). Efficacy of and patient compliance with a ketogenic diet in adults with intractable epilepsy: A meta-analysis. Journal of Clinical Neurology (Korea), 11(1), 26-31. https://doi.org/10.3988/jcn.2015.11.1.26
Yu, N., Han, F., Lin, X., Tang, C., Ye, J., & Cai, X. (2016). The Association Between Serum Selenium Levels with Rheumatoid Arthritis. Biological Trace Element Research, https://doi.org/10.1007/s12011-015-0558-2

## Claims

1. Medium chain triglycerides (MCT), optionally combined with dietary fibers, for use in therapeutic treatment of Rheumatoid arthritis (RA).

2. The MCT for use according to claim 1, wherein treatment of RA comprises inducing ketosis in a subject.

3. A composition suitable for human consumption comprising medium chain triglycerides (MCT), optionally combined with dietary fibers, for use in therapeutic treatment of Rheumatoid arthritis (RA).

4. The composition for use according to claim 3, wherein said composition is free of natural anti-inflammatory compounds chosen among epigallochatechin-3-gallate, curcumin and glucosinolates.

5. The composition for use according to claim 3 or 4, wherein said composition comprises said dietary fibers, wherein said dietary fibers comprise insoluble fibers and soluble fibers.

6. The composition for use according to claim 5, comprising an insoluble fibers to soluble fibers ratio of 1,5:1 to 3:1 wt/wt.

7. The composition for use according to any claims from 1 to 6, wherein said fibers include one or more of bamboo fiber, psyllium fiber, maize fiber.

8. The composition for use according to any of claims 3 to 7, wherein said composition contains MCT up to 100% wt/wt, in particular in a range of 2 to 80% wt/wt, in particular 5 to 60% wt/wt, more in particular 10 to 50% wt/wt, still more in particular 15 to 35% wt/wt.

9. The composition for use according to any of claims 5 to 8, comprising a MCT to dietary fibers ratio of 0,5:1 to 2:1, in particular of 0,75:1 to 1,5:1 wt/wt.

10. The composition for use according to any of claims 5 to 9, wherein said composition contains dietary fibers in a range of 2 to 50% wt/wt, in particular 5 to 45% wt/wt, more in particular 10 to 50% wt/wt, still more in particular 15 to 35% wt/wt.

11. The composition for use according to any of claims 3 to 10, wherein said MCT is provided as pure form or as a substance rich in MCT, in particular said MCT being in powder form or in liquid form.

12. The composition for use according to any of claims 3 to 11, wherein said MCT comprise a ratio of octanoic acid to decanoic acid of 50:50 to 70:30 wt/wt, in particular 55:45 to 65:35 wt/wt.

13. The composition for use according to any of claims 3 to 12, wherein said composition comprises fortifying food ingredients and/or micronutrients, in particular including one or more of omega 3, for example docosahexaenoic acid (DHA), eicosapentaenoic acid (EPA), minerals, trace elements, for example selenium, vitamins or a combination thereof.

14. The composition for use according to any of claims 3 to 13, wherein said composition comprises sweeteners and/or flavoring agents.

15. The composition for use according to any of claims 3 to 14, wherein said composition comprises proteins, carbohydrates, fibers and fat including said MCT.

16. The composition for use according to any of claims 3 to 15, wherein said composition essentially consists of proteins, carbohydrates, fibers, fat including said MCT and optionally one or more of fortifying food ingredients and/or micronutrients, sweeteners and/or flavoring agents.

17. The composition for use according to any of claims 3 to 12, wherein said composition essentially consists of fat including said MCT and optionally one or more of fortifying food ingredients and/or micronutrients, sweeteners and/or flavoring agents.

18. The composition for use according to any of claims 3 to 17 in the form of food in liquid or solid form, as powder product to be reconstituted with water, bars, bakery products, porridge, breakfast cereals, semi-solid products or liquid products.

19. The composition for use according to any of claims 3 to 18, wherein a dosage of at least 12g to 60g, in particular 15g to 45g MCT, and optionally of at least 10g to 60g, in particular 15g to 45g of dietary fibers, is administered per day.

20. A liquid composition suitable for human consumption comprising a composition according to any of claims 3 to 19 reconstituted with an aqueous liquid.

21. A bakery product comprising a composition according to any of claims 3 to 19.
